(19) Europäisches Patentamt

European Patent Office

Office européen des brevets

(11) Numéro de publication: **0 278 822 B1**

(12) **FASCICULE DE BREVET EUROPEEN**

(45) Date de publication du fascicule du brevet:
**23.05.90**

(21) Numéro de dépôt: **88400136.3**

(22) Date de dépôt: **22.01.88**

(51) Int. Cl.⁵: **C07C 313/02**, C07C 303/32, C07C 309/06

(54) Procédé de préparation de dérivés d'acides perhalogénoalcanesulfiniques et sulfoniques.

(30) Priorité: **04.02.87 FR 8701503**

(43) Date de publication de la demande:
**17.08.88 Bulletin 88/33**

(45) Mention de la délivrance du brevet:
**23.05.90 Bulletin 90/21**

(84) Etats contractants désignés:
**AT BE CH DE ES FR GB GR IT LI LU NL SE**

(56) Documents cités:
**Néant.**

(73) Titulaire: **RHONE-POULENC CHIMIE, 25, quai Paul Doumer, F-92408 Courbevoie Cédex(FR)**

(72) Inventeur: **Tordeux, Marc, 1, rue Seignelay, F-92330 Sceaux(FR)**
Inventeur: **Langlois, Bernard, 91, rue Duguesclin, F-69006 Lyon(FR)**
Inventeur: **Wakselman, Claude, 5, rue Chanez, F-75016 Paris(FR)**

(74) Mandataire: **Le Pennec, Magali et al, RHONE-POULENC INTERSERVICES Service Brevets Chimie 25, Quai Paul Doumer, F-92408 Courbevoie Cédex(FR)**

## Description

La présente invention concerne un procédé de préparation de dérivés d'acides perhalogénoalcanesulfiniques et sulfoniques. Elle concerne plus particulièrement la préparation de sels d'acides perfluoroalcanesulfiniques et sulfoniques.

Ces acides perfluoroalcanesulfoniques sont utilisés comme agents mouillants ou comme surfactants et détergence. Ils sont aussi utilisés dans la préparation de produits ayant des propriétés interfaciales.

Il est connu de préparer l'ensemble de ces composés par électrofluoration par exemple selon le brevet US 2 732 398. Ce procédé de préparation par voie électrochimique est exclu de par son prix de revient industriel trop élevé.

Parmi les méthodes chimiques il est connu de préparer ces composés par condensation du dioxyde de soufre sur des dérivés perfluoroalkylmagnésiens ou sur des iodures de perfluoroalkyle, selon le brevet US 4 221 734, en présence de zinc. Les dérivés magnésiens sont inutilisables d'un point de vue sécurité industrielle, car ils sont beaucoup trop violents. Le zinc présente l'inconvénient de donner naissance au cours du traitement du milieu réactionnel à de l'hydroxyde de zinc dont la séparation pose au niveau industriel de gros problèmes.

Il a maintenant été découvert un nouveau procédé permettant de surmonter l'inconvénient de l'art antérieur, et qui se présente du point de vue industriel sus un aspect économique particulièrement favorable.

La présente invention qui a permis d'atteindre cet objectif a pour objet un procédé de préparation de sels d'acides perhalogénoalcanesulfiniques de formule générale (I) :

$$\left| R_f - SO_2 \right|_{3-n} M .$$

dans laquelle, $R_f$ représente un groupe perhalogénoalkyle polyfluoré contenant au moins deux atomes de carbone et ne possédant pas un atome d'halogène autre que le fluor sur le carbone adjacent à celui qui porte le groupe $SO_2$, M représente un métal, n est égal à 1 ou 2 suivant l'état valentiel du métal M, caractérisé en ce qu'on met en présence dans un solvant polaire un hydroxyméthanesulfinate alcalin ou métallique de formule générale (II) :

$$M_n \left| SO_2 CH_2 OH \right|_{3-n} \quad (II)$$

dans laquelle n a la même signification que précédemment, et un halogénure de perhalogénoalkyle de formule : $R_fX$ dans laquelle $R_f$ représente un groupe perhalogénoalkyle polyfluoré contenant au moins deux atomes de carbone et ne possédant pas une atome d'halogène autre que le fluor sur le carbone adjacent à celui qui porte le groupe X qui représente le chlore, le brome ou l'iode.

Le procédé de la présente invention permet, contrairement à l'art antérieur précédemment cité et en particulier au brevet US 4 221 734 de préparer les acides de la présente invention ou leurs sels en l'absence de catalyseur métallique.

Parmi les composés de formule (I), on peut citer les sels de sodium, potassium de l'acide perfluorooctanesulfinique, de l'acide pentafluoroéthanesulfinique, de l'acide perfluorohexanesulfinique, de l'acide dichloro-1,1 trifluoro-2,2,2 éthanesulfinique.

Parmi les solvants polaires on préfère utiliser les amides tels que notamment : le formamide, le diméthylformamide, la N-méthylpyrrolidone, le diméthylacétamide et des solvants tels que le sulfolane. On préfère tout particulièrement utiliser le diméthylformamide.

Parmi les hydroxyméthanesulfinates on peut notamment citer l'hydroxyméthanesulfinate de sodium ou rongalite®, l'hydroxyméthanesulfinate de zinc ou décroline® et l'hydroxyméthanesulfinate de cuivre.

Parmi les halogénures de perhalogénoalkyle polyfluoré de formule $R_fX$ on préfère notamment utiliser ceux ayant 2 à 12 atomes de carbone.

Parmi la classe des halogénures de perfluoroalkyle, on préfère utiliser les iodures de perfluoroalkyle. En effet ces composés sont réputés pour leur inertie chimique et il est particulièrement surprenant qu'en présence d'hydroxyméthanesulfinates ils réagissent pour former des sels d'acides perfluoroalcanesulfiniques.

Selon un procédé préféré de mise en oeuvre de l'invention l'hydroxyméthanesulfinate alcalin ou métallique est introduit dans le réacteur, on préfère éliminer tout l'oxygène présent dans le réacteur puis on introduit l'halogénure de perhalogénoalkyle.

A la fin de la réaction on élimine le ou les solvants réactionnels, puis on purifie le sel de l'acide sulfinique obtenu par extraction à l'aide de solvants tels que l'acétate d'éthyle. On transforme ensuite par oxydation le sel de l'acide sulfinique en sel de l'acide sulfonique de manière connue par tout homme de l'art. On obtient alors l'acide par exemple par acidification du sel par l'acide sulfurique anhydre de manière elle aussi connue par tout homme de l'art.

L'oxydation peut être réalisée directement par introduction de chlore dans le milieu réactionnel, ce qui permet d'obtenir un chlorure de sulfonyle.

En ce qui concerne les conditions de réaction il est préférable de travailler à une température comprise entre 0° et 85°C encore plus préférentiellement à une température comprise entre 20 et 40°C.

De préférence le réacteur ne doit pas être constitué d'un matériau réactif tel que ceux décrits dans la demande de brevet publiée sous le numéro EP 165 135. On préfère ainsi utiliser un réacteur en verre.

Les acides perhalogénoalcanesulfoniques et leurs dérivés obtenus par le procédé de la présente invention sont utilisés comme catalyseurs dans des procédés comme l'alkylation ou comme agents tensio actifs notamment en détergence.

L'invention va être plus complètement décrite à l'aide des exemples suivants qui ne doivent pas être considérés comme limitatifs de l'invention.

## EXEMPLE 1 - PREPARATION DU DICHLORO-1,1 TRIFLUORO-2,2,2 ETHANESULFINATE DE SODIUM

Dans un erlenmeyer, on place 10 ml de diméthylformamide 3,7 g d'hydroxyméthanesulfinate de sodium et 3,74 g de trichloro-1,1,1 trifluoro-2,2,2 éthane1. Le mélange est agité pendant 12 h. Le taux de conversion en dichloro-1,1 trifluoro-2,2,2 éthanesulfinate de sodium ($\delta_F$ = -71 ppm) est de 30 % par rapport au trichlorotrifluoroéthane, et de 15 % par rapport à l'hydroxyméthanesulfinate de sodium.

## EXEMPLE II - PREPARATION DU CHLORURE DE PERFLUOROBUTANESULFONYLE

Dans un erlenmeyer, on place 10 ml de diméthylformamide, 2,6 g d'hydroxyméthanesulfinate de sodium et 3,5 g d'iodure de perfluorobutyle. Le mélange est agité pendant 12 heures. On constate par RMN du $F_{19}$ que 66 % de l'iodure est transformé. Après filtration, le diméthylformamide est évapore sous vide. On ajoute 20 ml d'eau. Les solides sont éliminés par filtration. On fait passer 1l de chlore à travers la solution 1,7 g de chlorure de perfluorobutanesulfonyle sont obtenus par décantion. Rendement 53 % (rendement 82 % par rapport à l'iodure de perfluoroalkyle transformé ($\delta_F$ = -104,6 ppm, Eb = 100°C, rendement par rapport à l'hydroxyméthanesulfinate de sodium : 26 %)

## EXEMPLE III - PREPARATION DU CHLORURE DE PERFLUOROOCTANESULFONYLE

a) On reproduit l'exemple 2 en replaçant l'iodure de perfluorobutyle par 5,5 g d'iodure de perfluorooctyle. 75 % de l'iodure est transformé. Après chloration, on obtient 3,2 g de chlorure de perfluorooctanesulfonyle. Rendement 62 % (82 % par rapport à l'iodure de perfluorooctyle transformé). $\delta_F$ = -104,6 ppm, F = 36°C, $Eb_{22mmHg}$ = 102°C.

b) L'essai précédent est répété avec 3,9 g d'hydroxyméthanesulfinate de zinc ; dans ce cas, l'iodure de perfluorooctyle est entièrement transformé. Après chloration, le rendement est de 85 %, et de 42 % par rapport à l'hydroxyméthanesulfinate de zinc.

## EXEMPLE IV - PREPARATION DE L'IODURE DE PERFLUOROOCTANESULFONYLE

Dans un erlenmeyer, on place 10 ml de diméthylformamide, 3,7 g d'hydroxyméthanesulfinate de sodium et 5,5 g d'iodure de perfluorooctyle. Le mélange est agité pendant 12 heures. On constate par resonance magnetique de fluor que 81 % de l'iodure est transformé, dont 24 % en hydrido-1 perfluorooctane $C_8F_{17}H$. On ajoute 5 ml d'eau ; le diméthylformamide est extrait au chlorure de méthylène, on fait passer 1 litre de chlore à travers la solution aqueuse et on obtient 2,5 g de chlorure de perfluorooctanesulfonyl. Rendement : 48 % (59 % par rapport à l'iodure de perfluorooctyle tranformé) et 24 % par rapport à l'hydroxyméthane sulfinate de sodium.

## Revendications

1°) Procédé de préparation de dérivés d'acides perhalogénoalcane-sulfiniques de formule générale (I) :

$$\left| R_f - SO_2 \right|_{3-n} M \qquad (I)$$

dans laquelle, $R_f$ représente un groupe perhalogénoalkyle polyfluoré contenant au moins deux atomes de carbone et ne possédant pas un atome d'halogène autre que le fluor sur le carbone adjacent au carbone qui porte le groups $SO_2$, M représente un métal, n est égal à 1 ou 2 suivant l'état valentiel du métal M, caractérisé en ce qu'on met en présence dans un solvant polaire du hydroxyméthanesulfinate alcalin ou métallique de formule générale (II) :

$$M_n \left| SO_2 CH_2 OH \right|_{3n} \qquad (II)$$

dans laquelle n a la même signification que précédemment, et un halogénure de perhalogénoalkyle de formule : $R_f X$ dans laquelle $R_f$ représente un groupe perhalogénoalkyle polyfluoré contenant au moins deux atomes de carbone et ne possédant pas un atome d'halogène autre que le fluor sur le carbone adjacent au carbone qui porte la groupe X et dans laquelle X représente le chlore, le brome ou l'iode.

2°) Procédé selon la revendication 1, caractérisé en ce que l'hydroxyméthanesulfinate est choisi parmi les sels de sodium, de zinc ou de cuivre.

3°) Procédé selon la revendication 1, caractérisé en ce que le solvant est le diméthylformamide.

4°) Procédé selon la revendication 1, caractérisé en ce que l'halogénure de perhalogénoalkyle est un iodure de perfluoroalkyle.

5°) Procédé selon la revendication 4, caractérisé en ce que l'iodure de perfluoralkyl contient de 2 à 12 atomes de carbone.

6°) Procédé selon la revendication 1, caractérisé en ce que la température réactionnelle est comprise entre 0° et 85°C et de préférence entre 20° et 40°C.

7°) Procédé de préparation de dérivés d'acides perhalogénoalcanesulfoniques, caractérisé en ce que dans une première étape on prépare un sel d'acide sulfinique selon l'une quelconque des revendications 1 à 6 et en ce que dans une 2ème étape, on oxyde le sel d'acide sulfinique en dérivé d'acide sulfonique.

## Claims

1. Process for the preparation of perhaloalkanesulphinic acid derivatives of general formula (I):

$$\left| R_f - SO_2 \right|_{3-n} M \qquad (I)$$

in which $R_f$ represents a polyfluorinated perhaloalkyl group containing at least two carbon atoms and not containing a halogen atom other than fluorine on the carbon atom adjacent to that which carries the SO2 group, M represents a metal, n is equal to 1 or 2 depending on the valency state of the metal M, characterized in that an alkali metal hydroxymethanesulphinate or a metal hydroxymethanesulphinate of general formula (II):

$$M_n \left| SO_2\, CH_2\, OH \right|_{3n} \qquad (II)$$

in which n has the same meaning as above is brought into contact, in a polar solvent, with a perhaloalkyl halide of formula: $R_fX$ in which $R_f$ represents a polyfluorinated perhaloalkyl group containing at least two carbon atoms and not containing a halogen atom other than fluorine on the carbon atom adjacent to that which carries the group X and in which X represents chlorine, bromine or iodine.

2. Process according to Claim 1, characterized in that the hydroxymethanesulphinate is chosen from amongst sodium, zinc or copper salts.

3. Process according to Claim 1, characterized in that the solvent is dimethylformamide.

4. Process according to Claim, 1, characterized in that the perhaloalkyl halide is a perfluoroalkyl iodide.

5. Process according to Claim 4, characterized in that the perfluoroalkyl iodide contains 2 to 12 carbon atoms.

6. Process according to Claim 1, characterized in that the reaction temperature is between 0° and 85°C and preferably between 20° and 40°C.

7. Process for the preparation of perhaloalkanesulphonic acid derivatives, characterized in that, in a first stage, a sulphinic acid salt is prepared according to any one of Claims 1 to 6 and in that, in a second stage, the sulphinic acid salt is oxidized into a sulphonic acid derivative.

**Patentansprüche**

1. Verfahren zur Herstellung von Derivaten der Perhalogenalkansulfinsäuren der allgemeinen Formel (I):

$$\left| R_f - SO_2 \right|_{3-n} M \qquad (I)$$

in der $R_f$ einen polyfluorierten Perhalogenalkylrest mit wenigstens zwei Kohlenstoffatomen darstellt, der kein anderes Halogenatom außer Fluor an den Kohlenstoff gebunden hat, der dem die SO2-Gruppe tragenden Kohlenstoff benachbart ist, in der M ein Metall darstellt, n gleich 1 oder 2 ist je nach Valenzzustand des Metalls M, dadurch gekennzeichnet, daß man in einem polaren Lösungsmittel ein Alkali- oder Metallhydroxymethansulfinat der allgemeinen Formel (II)

$$M_n \left| SO_2\, CH_2\, OH \right|_{3n} \qquad (II)$$

in der n die gleiche Bedeutung wie vorher hat, und ein Perhalogenalkylhalogenid der Formel $R_fX$ umsetzt, in der $R_f$ eine polyfluorierte Perhalogenalkylgruppe mit wenigstens zwei Kohlenstoffatomen darstellt, die kein Halogenatom außer Fluor an den Kohlenstoff gebunden hat, der dem Kohlenstoff gebunden hat, der dem Kohlenstoff, der die Gruppe X trägt, benachbart ist, und in der X Chlor, Brom oder Jod darstellt.

2. Verfahren nach Anspruch 1, dadurch gekennzeichnet, daß das Hydroxymethansulfinat ausgewählt wird aus den Salzen des Natriums, des Zinks oder des Kupfers.

3. Verfahren nach Anspruch 1, dadurch gekennzeichnet, daß das Lösungsmittel Dimethylformamid ist.

4. Verfahren nach Anspruch 1, dadurch gekennzeichnet, daß das Perhalogenalkylhalogenid ein Perfluoralkyljodid ist.

5. Verfahren nach Anspruch 4, dadurch gekennzeichnet, daß das Perfluoralkyljodid 2 bis 12 Kohlenstoffatomen enthält.

6. Verfahren nach Anspruch 1, dadurch gekennzeichnet, daß die Reaktionstemperatur zwischen 0° und 85°C und vorzugsweise zwischen 20° und 40°C liegt.

7. Verfahren zur Herstellung von Derivaten der Perhalogenalkansulfonsäuren, dadurch gekennzeichnet, daß man in einem ersten Schritt ein Sulfinsäuresalz gemäß einem der Ansprüche 1 bis 6 herstellt, und daß man in einem zweiten Schritt das Sulfinsäuresalz zu dem Sulfonsäurederivat oxidiert.